(19) Europäisches Patentamt
European Patent Office
Office européen des brevets

(11) EP 0 785 198 B1

(12) EUROPÄISCHE PATENTSCHRIFT

(45) Veröffentlichungstag und Bekanntmachung des Hinweises auf die Patenterteilung:
**05.07.2000 Patentblatt 2000/27**

(51) Int Cl.[7]: **C07D 453/02**

(21) Anmeldenummer: **96120280.1**

(22) Anmeldetag: **17.12.1996**

(54) **Verfahren zur Herstellung von optisch aktivem 3-Chinuclidinol**

Method for the preparation of optically active 3-hydroxyquinuclidine

Procédé pour la préparation de quinuclidine 3-hydroxy optiquement actif

(84) Benannte Vertragsstaaten:
**AT BE CH DE DK ES FI FR GB IE IT LI NL PT SE**

(30) Priorität: **19.01.1996 CH 13996**

(43) Veröffentlichungstag der Anmeldung:
**23.07.1997 Patentblatt 1997/30**

(73) Patentinhaber: **LONZA AG**
**CH-3945 Gampel/Wallis (CH)**

(72) Erfinder: **Brieden, Walter, Dr.**
**3902 Glis (Kanton Wallis) (CH)**

(74) Vertreter: **Weinhold, Peter, Dr.rer.nat.Dipl.Chem.**
**Winter, Brandl, Fürniss, Hübner,**
**Röss, Kaiser, Polte**
**Partnerschaft**
**Patent- und Rechtsanwaltskanzlei**
**Alois-Steinecker-Strasse 22**
**85354 Freising (DE)**

(56) Entgegenhaltungen:
**EP-A- 0 564 406** **EP-A- 0 577 253**
**EP-A- 0 577 394** **EP-A- 0 667 350**

- **CHEMICAL ABSTRACTS, vol. 92, no. 5, 4.Februar 1980 Columbus, Ohio, US; abstract no. 41726j, Seite 763; XP002030381 & ACTA PHARM. SUEC., Bd. 16, Nr. 4, 1979, Seiten 281-283, RINGDAHL ET AL.: "Facile preparation of the enantiomers of 3-acetoxyquinuclidine and 3-quinuclidinol"**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 785 198 B1

Printed by Jouve, 75001 PARIS (FR)

## Beschreibung

Die vorliegende Erfindung betrifft ein Verfahren zur Herstellung von optisch aktivem 3-Chinuclidinol der Formel

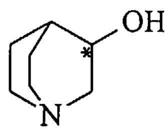

I.

Nichtracemische 1-Azabicyclo[2.2.2]octane mit Substituenten in der Position 3, wie 3-Chinuclidinol (I), sind Synthesebausteine für eine Reihe von pharmazeutischen Wirkstoffen (WO-A 93/06098, EP-A-0370415). Sie wurden bisher nahezu ausschliesslich durch Racematspaltung erhalten. Diese Trennung erfordert einen erheblichen Aufwand und liefert das unerwünschte Enantiomer als Abfall. Aufgabe der vorliegenden Erfindung war es, einen Weg zu nichtracemischem 3-Chinuclidinol zu eröffnen, der ohne Racematspaltung auskommt.

Erfindungsgemäss wird die Aufgabe durch das Verfahren nach Patentanspruch 1 gelöst.

Es wurde gefunden, dass sich das 3-Chinuclidinon der Formel

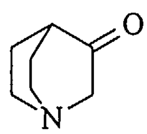

IIa,

dessen Addukte mit Lewis-Säuren und die entsprechenden tertiären und quartären Salze der allgemeinen Formel

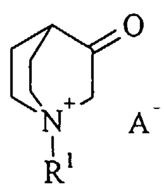

IIb,

worin $R^1$ Wasserstoff oder eine Mono-, Di- oder Triarylmethylgruppe und $A^-$ das Anion einer anorganischen oder organischen Säure ist, in Gegenwart eines katalytisch wirksamen optisch aktiven Komplexes von Rhodium, Iridium oder Ruthenium mit einem chiralen Diphosphin asymmetrisch hydrieren lassen. Die in den quartären Salzen (IIb, $R^1 \neq H$) gegebenenfalls vorhandenen Arylmethylgruppen können im gleichen oder in einem getrennten Hydrierungsschritt hydrogenolytisch abgespalten werden.

Unter Mono-, Di- und Triarylmethylgruppen sind hier und im folgenden Methylgruppen, die mit einer, zwei oder drei gleichen oder verschiedenen,substituierten oder unsubstituierten mono- oder polycyclischen aromatischen Gruppen substituiert sind, zu verstehen. Monocyclische aromatische Gruppen sind beispielsweise Phenyl, alkylierte Phenyle wie *o*-, *m*- und *p*-Tolyl oder die verschiedenen isomeren Xylyle, halogenierte Phenyle wie *o*-, *m*- und *p*-Chlorphenyl oder Bromphenyl, oder Alkoxyphenyle wie *o*-, *m*- und *p*-Methoxyphenyl. Polycyclische aromatische Gruppen sind beispielsweise 1- und 2-Naphthyl, Fluorenyle, Anthracenyle, Phenanthrenyle sowie die entsprechenden Alkyl-, Alkoxy- oder Halogenderivate.

Vorzugsweise wird das 3-Chinuclidinon als Addukt mit einer Lewis-Säure, insbesondere als Bortrifluorid-Addukt der Formel

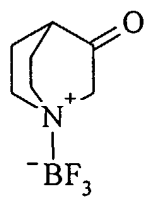

IIc

oder als Salz, insbesondere als quartäres Salz, eingesetzt. Als Salze kommen sowohl solche von anorganischen Säuren, wie beispielsweise die Chloride und Bromide, als auch solche mit organischen Säuren, wie beispielsweise die Acetate oder Mesylate, in Frage.

Besonders gute Resultate wurden mit Bromid als Anion und relativ voluminösen Substituenten $R^1$ wie beispielsweise o-Brombenzyl oder Diphenylmethyl erzielt.

Das 3-Chinuclidinon-Hydrochlorid ist kommerziell erhältlich, die freie Base kann daraus sehr einfach durch Umsetzen mit einer starken Base wie beispielsweise Natriumhydroxid und anschliessende Extraktion erhalten werden. Die Addukte mit Lewis-Säuren können durch einfache Zugabe der Lewis-Säure, also beispielsweise Bortrifluorid oder Bortrifluorid-Etherat, zu einer Lösung von 3-Chinuclidinon hergestellt werden. Die tertiären Salze sind entsprechend durch Zugabe einer (BrØnsted-) Säure erhältlich. Die quartären Salze können auf übliche Weise durch Umsetzen des 3-Chinuclidinons mit einem Alkylierungsmittel wie beispielsweise Benzylchlorid, -bromid oder Diphenylmethylbromid und gegebenenfalls anschliessenden Anionenaustausch hergestellt werden.

Als katalytisch wirksame optisch aktive Komplexe werden vorzugsweise Rhodiumkomplexe eingesetzt. Diese werden vorteilhaft *in situ* aus einem geeigneten Vorläuferkomplex und dem chiralen Diphosphin hergestellt.

Als Vorläuferkomplex wird vorzugsweise ein Olefinkomplex eingesetzt. Besonders bevorzugt ist das kommerziell erhältliche Bis-(1,5-cyclooctadien)-dirhodium(I)-dichlorid ($[Rh(COD)Cl]_2$), das vorteilhaft im molaren Verhältnis 1:2 mit dem chiralen Diphosphin umgesetzt wird.

Als chirale Diphosphin-Liganden eignen sich beispielsweise 1,4-Bis(diphenylphosphino)-1,4-dideoxy-2,3-O-isopropylidenthreitol ("DIOP"), 2,2'-Bis(diphenylphosphino)-1,1'-binaphthalin ("BINAP"), (*R**,*R**)-4-Diphenylphosphino-2-(diphenylphosphinomethyl)-pyrrolidin ("PPM") oder (*R**,*R**)-2,3-Bis-(diphenylphosphino)-butan ("Chiraphos"). Diese Liganden sind kommerziell erhältlich, beispielsweise bei der Firma Fluka.

Besonders bevorzugt sind chirale Diphosphine mit Metallocenstruktur wie beispielsweise die disubstituierten Ferrocene der allgemeinen Formel

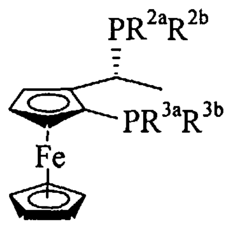

III,

worin $R^{2a}$, $R^{2b}$, $R^{3a}$ und $R^{3b}$ unabhängig voneinander jeweils $C_{1-12}$-Alkyl, $C_{5-7}$-Cydoalkyl oder gegebenenfalls substituiertes Phenyl bedeuten, sowie deren Spiegelbild.

Ganz besonders bvorzugt sind das (*R*)-1-[(*S*)-2-(Diphenylphosphino)ferrocenyl]ethyl-di-tert-butylphosphin ($R^{2a}$ = $R^{2b}$ = tert-Butyl, $R^{3a}$ = $R^{3b}$ = Phenyl, IIIa) und das (*R*)-1-[(*S*)-2-(Diphenylphosphino)ferrocenyl]ethyl-dicyclohexylphosphin ($R^{2a}$ = $R^{2b}$ = Cyclohexyl, $R^{3a}$ = $R^{3b}$ = Phenyl, IIIb) sowie deren Spiegelbilder. Die Herstellung dieser Liganden ist in EP-A-0 564 406 beschrieben. Mit diesen Liganden wurden auch bei hohen molaren Verhältnissen Edukt/Katalysator in der Grössenordnung $10^3$ gute optische Ausbeuten von bis zu 60% ee erreicht.

Die Hydrierung wird vorteilhaft bei einer Temperatur von 0 bis 100 °C und einem Wasserstoffdruck von 1 bis 150 bar durchgeführt.

Als Lösungsmittel eignen sich beispielsweise niedere Alkohole wie Methanol, Ester wie Ethylacetat, Ketone wie Aceton oder aromatische Kohlenwasserstoffe wie Toluol.

Die folgenden Beispiele verdeutlichen die Durchführung des erfindungsgemässen Verfahrens.

**Beispiel 1:**

**3-Chinuclidinon**

Zu einer Lösung von 300 g (7,50 mol) Natriumhydroxid in 1 l Wasser wurde bei 40 °C 300 g (1,856 mol) 3-Chinuclidinon-hydrochlorid (Fluka) gegeben und die entstandene Lösung auf 20 °C abgekühlt. Anschliessend wurde dreimal mit je 400 ml Ethylacetat extrahiert und die vereinigten organischen Extrakte über Magnesiumsulfat getrocknet. Nach dem Eindampfen wurden 220 g (95%) der Titelverbindung als hellbeiger Feststoff erhalten.

| | | |
|---|---|---|
| Schmp.: | | 146-147 °C |
| $^1H$ NMR ($CDCl_3$, 300 MHz): | δ = | 3,39-3,19 (m, 2H); |
| | | 3,10-2,81 (m, 4H); |
| | | 2,50-2,43 (m, 1H); |
| | | 2,10-1,90 (m, 4H). |

**Beispiel 2:**

**3-Chinuclidinon-hydrobromid**

Zu einer Lösung von 19,0 g (152 mmol) 3-Chinuclidinon in 50 ml Wasser wurde 21,8 g (167 mmol) 62%ige Bromwasserstoffsäure getropft und das Reaktionsgemisch zur Trockne eingedampft. Der Rückstand wurde mit 100 ml Tetrahydrofuran aufgeschlämmt, abgefrittet und mit 50 ml Tetrahydrofuran gewaschen. Nach dem Trocknen wurden 30,5 g (98%) der Titelverbindung als weisse Kristalle isoliert.

**Beispiel 3:**

**3-Chinuclidinon-Bortrifluorid-Addukt**

Zu 33,2 g (266 mmol) 3-Chinuclidinon in 100 ml Diethylether und 250 ml *n*-Hexan wurde 36,5 g (257 mmol) Bortrifluorid-etherat innerhalb von 40 min getropft. Der ausgefallene Feststoff wurde nach 1 h abgefrittet und ergab nach dem Trocknen 50,2 g (98%) der Titelverbindung als weisses Pulver.

NMR-Daten: Die Spektren wurden in $d_4$-Methanol aufgenommen. Durch reversible Addition eines Lösungsmittelmoleküls an die Carbonylgruppe bildet sich ein Halbacetal, dessen Signale im folgenden angegeben sind.

| | | |
|---|---|---|
| $^1H$ NMR (400 MHz) | δ = | 4,00-3,98 (m, 2H); |
| | | 3,63-3,52 (m, 2H); |
| | | 3,50-3,42 (m, 2H); |
| | | 2,74-2,70 (m, 1H); |
| | | 2,37-2,27 (m, 2H); |
| | | 2,24-2,13 (m, 2H). |

| | | |
|---|---|---|
| $^{13}C$ NMR (100 MHz) | δ = | 96,42 (s); |
| | | 61,07 (t); |
| | | 47,53 (t); |
| | | 47,23 (t); |
| | | 30,71 (d); |
| | | 20,38 (t); |
| | | 20,05 (t). |

(Angegeben sind nur die Multiplizitäten, die sich durch direkte $^1H$-$^{13}C$-Kopplungen ergeben.)

**Beispiel 4:**

**1-Benzyl-3-oxochinuclidiniumchlorid**

Zu 61,6 g (492 mmol) 3-Chinuclidinon in 250 ml Acetonitril wurde 62,3 g (492 mmol) Benzylchlorid innerhalb von 30 min getropft und die entstandene Suspension nach 2 h bei 25 °C abgefrittet. Waschen mit Acetonitril (2×100

ml) und Trocknen ergab 115,0 g (93%) der Titelverbindung als weissen, kristallinen Feststoff.

$^1$H NMR (DMSO-$d_6$, 400 MHz): δ = 7,62-7,56 (m, 2H); 7,55-7,50 (m, 3H); 4,75 (s, 2H); 4,29 (s, 2H); 3,78-3,60 (m, 4H); 2,70-2,66 (m, 1H); 2,29-2,40 (m, 2H); 2,12-2,02 (m, 2H).

**Beispiel 5:**

**1-Benzyl-3-oxochinuclidiniumbromid**

Zu 29,5 g (236 mmol) 3-Chinuclidinon in 120 ml Acetonitril wurde 40,4 g (236 mmol) Benzylbromid innerhalb von 15 min getropft und die entstandene Suspension nach 2 h bei 25 °C abgefrittet. Waschen mit *n*-Hexan (2×50 ml) und Trocknen ergab 65,3 g (93%) der Titelverbindung als weissen, kristallinen Feststoff.

$^1$H NMR (DMSO-$d_6$, 400 MHz): δ = 7,56-7,50 (m, 3H); 7,56-7,50 (m, 3H); 4,82 (s, 2H); 4,34 (s, 2H); 3,82-3,73 (m, 2H); 3,73-3,64 (m, 2H); 2,71-2,67 (m, 1H); 2,32-2,22 (m, 2H); 2,13-2,01 (m, 2H).

$^{13}$C NMR (DMSO-$d_6$, 100 MHz): δ = 202,49; 133,01; 130,28; 128,96; 127,08; 65,92; 64,42; 53,61; 37,29; 20,65.

**Beispiel 6:**

**1-(4-Methoxybenzyl)-3-oxochinuclidiniumbromid**

Zu 1,25 g (10,0 mmol) 3-Chinuclidinon in 10 ml Acetonitril wurde 1,57 g (10,0 mmol) 4-Methoxybenzylchlorid gegeben und die entstandene Suspension nach 30 min abgefrittet. Waschen mit *n*-Hexan und Trocknen ergab 2,33 g (83%) der Titelverbindung als weissen, kristallinen Feststoff.

$^1$H NMR ($CDCl_3$, 400 MHz): δ = 7,65-7,61 ("d", 2H); 6,95-6,91 ("d", 2H); 5,23 (s, 2H); 4,66 (s, 2H); 4,19-4,10 (m, 2H); 4,08-3,98 (m, 2H); 3,82 (m, 3H); 2,79-2,75 (m, 1H); 2,40-2,30 (m, 2H); 2,28-2,17 (m, 2H).

**Beispiel 7:**

**1-(Diphenylmethyl)-3-oxochinuclidiniumbromid**

Zu 12,5 g (100 mmol) 3-Chinuclidinon in 100 ml Acetonitril wurde 26,0 g (100 mmol) Bromdiphenylmethan (~95%) unter Rühren gegeben und die entstandene Suspension nach 24 h abgefrittet. Waschen mit Diethylether (100 ml) und Trocknen ergab 32,0 g (86%) der Titelverbindung als weissen, kristallinen Feststoff.

| | | |
|---|---|---|
| $^1$H NMR ($CDCl_3$, 400 MHz): | $\delta$ = | 7,99-7,94 (m, 4H); |
| | | 7,57-7,46 (m, 6H); |
| | | 6,32 (s, 1H); |
| | | 4,28 (s, 2H); |
| | | 3,83-3,65 (m, 4H); |
| | | 2,69-2,66 (m, 1H); |
| | | 2,30-2,20 (m, 2H); |
| | | 2,13-2,03 (m, 2H). |

**Beispiel 8:**

**(*S*)-3-Chinuclidinol**

Im Autoklav (450 ml) wurden 30,0 g (186 mmol) 3-Chinuclidinon-hydrochlorid, 18,3 mg (0,0371 mmol) Bis-(1,5-cyclooctadien)-dirhodium(I)-dichlorid, 40,3 mg (0,0743 mmol) (*R*)-1-[(*S*)-2-(Diphenylphosphino)ferrocenyl]ethyl-di-*tert*-butylphosphin (IIIa, entsprechend einem molaren Verhältnis Edukt/Katalysator = 2500) und 300 ml entgastes Methanol unter Argon eingefüllt. Bei 70 °C und 50 bar $H_2$ wurde 19 h hydriert. Das Reaktionsgemisch wurde eingedampft und der Rückstand in 250 ml 2 Salzsäure gelöst. Nach zweimaliger Extraktion mit je 40 ml Dichlormethan wurde mit Natronlauge (30%) auf pH = 14 gestellt und eingedampft. Der Rückstand wurde mit je 350 ml Ethylacetat unter Rückfluss erhitzt und heiss abfiltriert. Die wässrige Phase wurde abgetrennt und die organische eingedampft. Umkristallisation aus Ethylacetat (150 ml) ergab 22,3 g (94%) (*S*)-3-Chinuclidinol mit 24% ee.
$[\alpha]_D^{25}$ = +11,0 (*c* = 2, 1 HCl)

**Beispiel 9:**

**(*R*)-3-Chinuclidinol**

In einen Autoklaven (1 l) wurden 100 g (268 mmol) 1-(Diphenylmethyl)-3-oxochinuclidiniumbromid, 66 mg (0,133 mmol) Bis-(1,5-cyclooctadien)-dirhodium(I)-dichlorid, 145 mg (0,267 mmol) (*S*)-1-[(*R*)-2-(Diphenylphosphino) ferrocenyl]ethyl-di-*tert*-butylphosphin (IIIa, Spiegelbild; entsprechend einem molaren Verhältnis Edukt/Katalysator = 1000) und 500 ml entgastes Methanol unter Argon eingefüllt. Bei 75 °C und 10 bar $H_2$ wurde 19 h hydriert. Nach dem Abkühlen wurde 3 g Pd/C (5% Pd) in 50 ml Methanol zugegeben und bei 75 °C und 10-14 bar $H_2$ wurde 4 h weiter hydriert. Anschliessend wurde abgekühlt, filtriert und eingedampft. Der Rückstand wurde mit 200 ml Wasser und 100 ml Ethylacetat aufgenommen und die wässrige Phase noch zweimal mit je 50 ml Ethylacetat gewaschen. Die wässrige Phase wurde mit Natronlauge (30%) auf pH = 14 gestellt und zur Trockne eingedampft. Der Rückstand wurde viermal mit je 100 ml Dichlormethan extraktiv behandelt und die vereinigten organischen Extrakte über $MgSO_4$ getrocknet und eingedampft. Es wurden 33,0 g (96,6%) der rohen Titelverbindung als weisser kristalliner Feststoff erhalten. Umkristallisation aus Ethylacetat ergab 28,3 g (83%) (*R*)-3-Chinuclidinol mit 58% ee.
$[\alpha]_D^{25}$ = —26,1 (*c* = 2, 1 HCl)

**Beispiel 10:**

**(*S*)-3-Chinuclidinol**

Im Autoklav (160 ml) wurden 5,00 g (40 mmol) 3-Chinuclidinon unter Argon mit einer Lösung von 200 mg (0,41 mmol) Bis-(1,5-cyclooctadien)-dirhodium(I)-dichlorid und 480 mg (0,81 mmol) (*R*)-1-[(*S*)-2-(Diphenylphosphino) ferrocenyl]ethyl-dicyclohexylphosphin (IIIb, entsprechend einem molaren Verhältnis Edukt/Katalysator = 50) in 70 ml entgastem Methanol versetzt. Bei 75 °C und 50 bar $H_2$ wurde 22 h hydriert. Nach dem Eindampfen der Reaktionslösung wurde mit 1 Salzsäure auf pH = 1 gestellt und dreimal mit je 50 ml Dichlormethan extrahiert. Die wässrige Phase wurde mit Natronlauge (30%) auf pH = 14 gestellt und zur Trockne eingedampft. Der Rückstand wurde viermal mit je 100 ml

Dichlormethan extrahiert und die vereinigten organischen Extrakte über $MgSO_4$ getrocknet und eingedampft. Es wurden 3,40 g (67%) der rohen Titelverbindung als weisser kristalliner Feststoff erhalten. Umkristallisation aus Ethylacetat ergab 2,65 g (52%) (*S*)-3-Chinuclidinol mit 7% ee.
$[\alpha]_D^{25}$ = +3,2 (*c* = 2, 1 HCl)

$^1$H NMR ($CD_3OD$, 400 MHz): $\delta$ = 3,87-3,82 (m, 1H); 3,13-3,05 ("ddd", 1H);
2,90-2,81 (m, 1H);
2,80-2,70 (m, 2H);
2,69-2,60 (m, 1H);
2,58-2,51 ("dt", 1H);
2,02-1,92 (m, 1H);
1,83-1,77 (m, 1H);
1,76-1,68 (m, 1H);
1,58-1,47 (m, 1H);
1,45-1,36 (m, 1H).

$^{13}$C NMR ($CD_3OD$, 100 MHz): $\delta$ = 68,16; 58,17;
48,13;
47,06;
29,08;
25,28;
19,62.

**Beispiele 11-28:**

Diese Beispiele wurden analog zu den Beispielen 8-10 mit $[Rh(COD)Cl]_2$ als Vorläuferkomplex durchgeführt. Die Ergebnisse sind in der folgenden Tabelle 1 zusammengefasst. Angegeben ist jeweils das Edukt mit Formelnummer und gegebenenfalls den variablen Substituenten, der chirale Diphosphinligand mit seiner absoluten Konfiguration (IIIa = (*R*)-1-[(*S*)-2-(Diphenylphosphino)ferrocenyl]ethyl-di-*tert*-butylphosphin, IIIb = (*R*)-1-[(*S*)-2-(Diphenylphosphino)ferrocenyl]ethyl-dicyclohexylphosphin), das Lösungsmittel, das molare Verhältnis Edukt/Katalysator (E/C), die spezifische Drehung des Produkts, sowie dessen optische Reinheit (als ee-Wert) und die Konfiguration des bevorzugt gebildeten Enantiomeren.

Tabelle 1

| Edukt | Ligand | Lösungsmittel | E/C | $[\alpha]_D$ | ee (Konfig.) |
|---|---|---|---|---|---|
| IIa | IIIa | Toluol | 50 | +0,4 | <1% (*S*) |
| IId | IIIa | MeOH | 50 | +5,3 | 12% (*S*) |
| IId | IIIb | MeOH | 50 | +8,1 | 18% (*S*) |
| IId | IIIa | EtOAc | 500 | +0,5 | <1% (*S*) |
| IIb ($R^1$= $C_6H_5CH_2$, $A^-$= $Cl^-$) | IIIa | MeOH | 2500 | +14,1 | 31% (*S*) |
| IIb ($R^1$ = $C_6H_5CH_2$, $A^-$ = $Br^-$) | IIIa | MeOH | 2500 | +22,4 | 50% (*S*) |
| IIb ($R^1$ = $C_6H_5CH_2$, $A^-$ = $Br^-$) | IIIb | MeOH | 250 | +16,9 | 38% (*S*) |
| IIb ($R^1$ = *o*-$BrC_6H_4CH_2$, $A^-$ = $Br^-$) | IIIa | MeOH | 1000 | +23,9 | 53% (*S*) |
| IIb ($R^1$ = H, $A^-$ = $Cl^-$) | IIIb | MeOH | 1550 | +12,1 | 27% (*S*) |
| IIb ($R^1$ = H, $A^-$ = $Cl^-$) | IIIa | MeOH | 5000 | +10,7 | 24% (*S*) |
| IIb ($R^1$ = H, $A^-$ = $Br^-$) | IIIa | MeOH | 2500 | +12,9 | 28% (S) |
| IIb ($R^1$ = H, $A^-$ = $Cl^-$) | (*S*)-PPM | MeOH | 50 | -1,8 | 4% (*R*) |
| IIb ($R^1$ = H, $A^-$ = $Cl^-$) | (*R,R*)-DIOP | MeOH | 50 | +0,8 | 2% (*S*) |
| IIb ($R^1$ = H, $A^-$ = $Cl^-$) | (*R*)-BINAP | MeOH | 50 | +0,3 | <1% (*S*) |
| IIb ($R^1$ = H, $A^-$ = $Cl^-$) | (*S*)-Chiraphos | MeOH | 50 | -4,2 | 9% (*R*) |

Tabelle 1 (fortgesetzt)

| Edukt | Ligand | Lösungsmittel | E/C | $[\alpha]_D$ | ee (Konfig.) |
|---|---|---|---|---|---|
| IIb ($R^1$ = H, $A^-$ = $OAc^-$) | IIIa | MeOH | 1000 | +0,7 | 1% (*S*) |
| IIb ($R^1$ = H, $A^-$ = $MeSO_3^-$) | IIIa | MeOH | 1000 | +2,0 | 4% (*S*) |
| IIb ($R^1$ = $CHPh_2$, $A^-$ = $Br^-$) | IIIb | MeOH | 2500 | +26,8 | 60% (*S*) |

## Patentansprüche

**1.** Verfahren zur Herstellung von optisch aktivem 3-Chinuclidinol der Formel

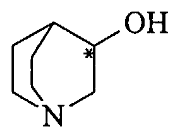

I,

dadurch gekennzeichnet, dass ein Chinuclidinderivat aus der Gruppe bestehend aus 3-Chinuclidinon der Formel

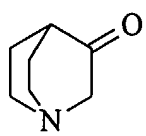

IIa

und dessen Addukten mit Lewis-Säuren,
sowie den entsprechenden tertiären und quartären Salze der allgemeinen Formel

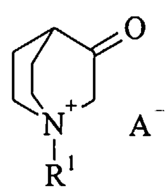

IIb,

worin $R^1$ Wasserstoff oder eine Mono-, Di- oder Triarylmethylgruppe und $A^-$ das Anion einer anorganischen oder organischen Säure ist,
in Gegenwart eines katalytisch wirksamen optisch aktiven Komplexes von Rhodium, Iridium oder Ruthenium mit einem chiralen Diphosphin als Liganden asymmetrisch hydriert und gegebenenfalls der Substituent $R^1$ abgespalten wird.

**2.** Verfahren nach Anspruch 1, dadurch gekennzeichnet, dass als Addukt des 3-Chinuclidinons (IIa) mit einer Lewis-Säure das Bortrifluorid-Addukt der Formel

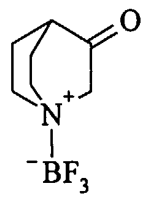

IIc

eingesetzt wird.

**3.** Verfahren nach Anspruch 1, dadurch gekennzeichnet, dass als Salz des 3-Chinuclidinons ein quartäres Salz, worin $R^1$ *o*-Brombenzyl oder Diphenylmethyl ist, eingesetzt wird.

**4.** Verfahren nach Anspruch 3, dadurch gekennzeichnet, dass als quartäres Salz ein Bromid eingesetzt wird.

**5.** Verfahren nach einem der Ansprüche 1 bis 4, dadurch gekennzeichnet, dass als katalytisch wirksamer optisch aktiver Komplex ein Rhodiumkomplex eingesetzt wird.

**6.** Verfahren nach einem der Ansprüche 1 bis 5, dadurch gekennzeichnet, dass als chirales Diphosphin ein Diphosphin mit Metallocenstruktur der allgemeinen Formel

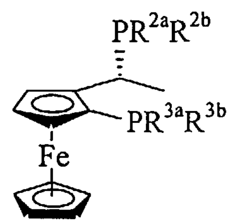

III,

worin $R^{2a}$, $R^{2b}$, $R^{3a}$ und $R^{3b}$ unabhängig voneinander jeweils $C_{1-12}$-Alkyl, $C_{5-7}$-Cycloalkyl oder gegebenenfalls substituiertes Phenyl bedeuten, oder dessen Spiegelbild eingesetzt wird.

**7.** Verfahren nach Anspruch 6, dadurch gekennzeichnet, dass als Diphosphin mit Metallocenstruktur (III) ein Diphosphin eingesetzt wird, worin $R^{3a} = R^{3b}$ = Phenyl und $R^{2a} = R^{2b}$ = *tert*-Butyl oder $R^{2a} = R^{2b}$ = Cyclohexyl ist.

**Claims**

**1.** Process for the preparation of optically active 3-quinuclidinol of the formula

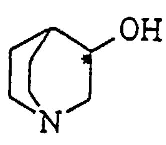

I,

characterised in that a quinuclidine derivative from the group consisting of 3-quinuclidinone of the formula

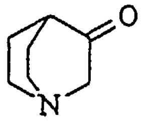

IIa

and its adducts with Lewis acids,
and the corresponding tertiary and quaternary salts of the general formula

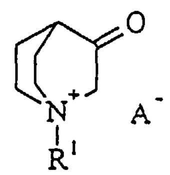

IIb,

wherein $R^1$ is hydrogen or a mono-, di- or triarylmethyl group and $A^-$ is the anion of an inorganic or organic acid, is hydrogenated asymmetrically in the presence of a catalytically active optically active complex of rhodium, iridium or ruthenium with a chiral diphosphine as ligand, and optionally the substituent $R^1$ is removed.

2. Process according to claim 1, characterised in that there is used as the adduct of 3-quinuclidinone (IIa) with a Lewis acid the boron trifluoride adduct of the formula

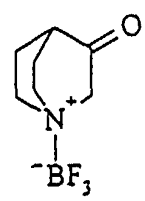

IIc

3. Process according to claim 1, characterised in that there is used as the salt of 3-quinuclidinone a quaternary salt wherein $R^1$ is o-bromobenzyl or diphenylmethyl.

4. Process according to claim 3, characterised in that a bromide is used as the quaternary salt.

5. Process according to any one of claims 1 to 4, characterised in that a rhodium complex is used as the catalytically active optically active complex.

6. Process according to any one of claims 1 to 5, characterised in that there is used as the chiral diphosphine a diphosphine having a metallocene structure of the general formula

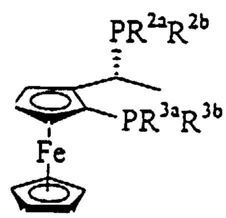

III.

wherein $R^{2a}$, $R^{2b}$, $R^{3a}$ and $R^{3b}$ are each independently of the others $C_{1-12}$-alkyl, $C_{5-7}$-cycloalkyl or optionally substituted phenyl, or the mirror image thereof.

7. Process according to claim 6, characterised in that there is used as the diphosphine having a metallocene structure (III) a diphosphine wherein $R^{3a} = R^{3b}$ = phenyl and $R^{2a} = R^{2b}$ = tert-butyl or $R^{2a} = R^{2b}$ = cyclohexyl.

## Revendications

1. Procédé pour la préparation de 3-quinuclidine activée optiquement de la formule

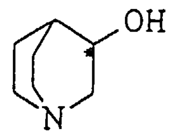

I,

caractérisé en ce qu'un dérivé de quinuclidine du groupe constitué par la 3-quinuclidinone de la formule

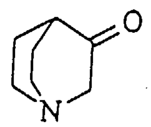

IIa

et de ses produits d'addition avec des acides de Lewis ainsi que de leurs sels tertiaires et quaternaires correspondants de la formule générale

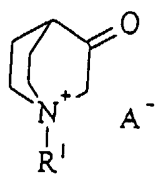

IIb,

dans laquelle $R^1$ est l'hydrogène ou un groupe mono, di ou triarylméthyle et $A^-$ est l'anion d'un acide minéral ou organique,
est hydrogénaté en présence d'un complexe actif optiquement et catalytiquement efficace de rhodium, iridium ou ruthénium avec une diphosphine chirale en tant que ligands asymétriquement et le substituant $R^1$ est éventuellement séparé.

2. Procédé selon la revendication 1, caractérisé en ce qu'en tant que produit d'addition de la 3-quinuclidinone (IIa) on met en oeuvre les produits d'addition borotrifluorure avec un acide de Lewis selon la formule

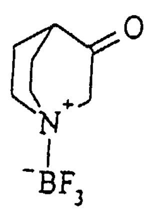

IIc

3. Procédé selon la revendication 1, caractérisé en ce qu'en tant que sel de la 3-quinuclidinone on met en oeuvre un sel quaternaire, moyennant quoi $R^1$ est o-brombenzyle ou diphénylméthyle.

4. Procédé selon la revendication 3, caractérisé en ce qu'en tant que sel quaternaire on met en oeuvre un bromure.

5. Procédé selon l'une des revendications 1 à 4, caractérisé en ce qu'en tant que complexe actif optiquement et catalytiquement efficace on met en oeuvre un complexe de rhodium.

6. Procédé selon l'une des revendications 1 à 5, caractérisé en ce qu'en tant que diphosphine chirale on met en

oeuvre une diphosphine avec une structure métallocène de la formule générale

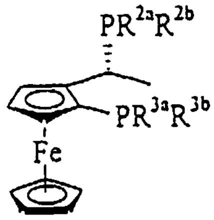

III,

dans laquelle $R^{2a}$, $R^{2b}$, $R^{3a}$ et $R^{3b}$ signifient indépendamment l'un de l'autre chaque fois alkyle $C_{1-12}$, cycloalkyle $C_{5-7}$ ou éventuellement un phényle substitué ou son image spéculaire.

**7.** Procédé selon la revendication 6, caractérisé en ce qu'en tant que diphosphine avec une structure métallocène (III) on met en oeuvre une diphosphine, moyennant quoi $R^{3a}$=$R^{3b}$= phényle et $R^{2a}$=$R^{2b}$= tert-butyle ou $R^{2a}$=$R^{2b}$ =cyclohexyle.